# EUROPÄISCHE PATENTSCHRIFT

(11) **EP 0 583 637 B1**
(45) Veröffentlichungstag und Bekanntmachung des Hinweises auf die Patenterteilung: **12.03.1997**
(21) Anmeldenummer: 93111624.8
(22) Anmeldetag: 21.07.1993
(51) Int. Cl.: C07C 263/06

(54) **Verfahren zur Herstellung von Isocyanaten durch Zersetzen von N,N,N'-trisubstituierten Harnstoffen**
Process for the preparation of isocyanates by decomposition of N,N,N'-trisubstituted ureas
Procédé pour la préparation d'isocyanates par décomposition d'urées N,N,N'-substituées

(30) Priorität: 13.08.1992 AT 1631/92
(43) Veröffentlichungstag der Anmeldung: 23.02.1994
(73) Patentinhaber: DSM Chemie Linz GmbH, 4021 Linz (AT)
(72) Erfinder: Hackl, Kurt Alfred, Dipl.-Ing.Dr., A-4020 Linz (AT); Falk, Heinz, Prof.Dr., (AT)
(74) Vertreter: Kunz, Ekkehard, Dr.

(56) Entgegenhaltungen:
- CH-A- 228 816
- FR-A- 1 473 821
- US-A- 3 936 484
- US-A- 4 141 913

## Beschreibung

Die Erfindung betrifft ein Verfahren zur Herstellung von Isocyanaten durch Zersetzen von N,N,N'-trisubstituierten Harnstoffen.

Isocyanate stellen eine wirtschaftlich bedeutende Gruppe von Zwischenprodukten dar, die unter anderem in der Kunststoffindustrie, in der Produktion von Lacken und in der Pflanzenschutzmittelherstellung eingesetzt werden.
Aus diesem Grund ist auch eine Vielzahl von Verfahren zur Herstellung von Isocyanaten bekannt, die aber mehrere Nachteile aufweisen. So gehen zum Beispiel ältere Verfahren hauptsächlich von Phosgen oder Phosgenfolgeprodukten aus, während neuere Verfahren, die den Einsatz von Phosgen vermeiden, Isocyanate nur in schlechten Ausbeuten erzielen.
Es wurden auch schon Verfahren beschrieben, die von substituierten Harnstoffen, die thermisch zersetzt werden, ausgehen.
In der französischen Patentschrift 1,473,821 werden trisubstituierte Harnstoffe in Isocyansäureester und Amine, bei Temperaturen von unter 200°C in einem Lösungsmittel mit einer Dielektrizitätskonstante unter 40, zersetzt. Die Isocyanate werden jedoch nur in einer Ausbeute von etwa 60 % erhalten.
Die US-Patentschrift 3,936,484 schlägt ein Verfahren zur thermischen Zersetzung disubstituierter Harnstoffe, insbesondere zur Herstellung von Toluylen-2,4-diisocyanat, vor. Bevorzugt wird das entstehende Amin mittels eines Trägergasstromes, dem ein zusätzliches Lösungsmittel zugesetzt werden kann, aus dem Reaktionsgemisch entfernt.

Die Nachteile dieses Verfahrens sind jedoch, daß hauptsächlich nur das entstehende Amin über Kopf abgezogen werden kann und das eigentliche Produkt im Sumpf bleibt und anschließend isoliert und gereinigt werden muß, und daß nur durch Zugabe eines zusätzlichen Lösungsmittels eine gute Ausbeute erzielt wird.

Es konnte nun unerwarteterweise ein Verfahren zur Herstellung von Isocyanaten gefunden werden, das von bestimmten N,N,N'-tri-substituierten Harnstoffen ausgeht, ohne den Einsatz von Phosgen auskommt und die gewünschten Isocyanate in einfacher Weise, hoher Ausbeute und Reinheit zugänglich macht.

Gegenstand der Erfindung ist demnach ein Verfahren zur Herstellung von Isocyanaten, dadurch gekennzeichnet, daß N,N,N'-trisubstituierte Harnstoffe der Formel 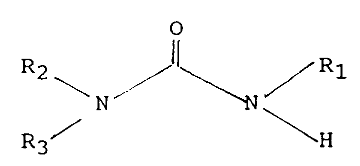 in der R₁ einen geradkettigen, verzweigten oder cyclischen (C₂ - C₁₀) Alkylrest oder einen Benzylrest und R₂ und R₃ einen geradkettigen, verzweigten oder cyclischen (C₄ - C₂₀) Alkylrest oder einen Benzylrest bedeuten, in einem unter Reaktionsbedingungen inerten Verdünnungsmittel bei erhöhter Temperatur in ein leichtflüchtiges Isocyanat der Formel R₁NCO und in ein schwer flüchtiges sekundäres Amin der Formel R₂R₃NH, dessen Siedepunkt über dem des Isocyanates und über der angewandten Reaktionstemperatur liegt, zersetzt werden, wobei das Isocyanat mittels eines Trägergasstromes über Kopf abgezogen wird.

Als Ausgangsverbindungen eignen sich prinzipiell alle N,N,N'-trisubstituierten Harnstoffe, die bei thermischer Belastung in ein leichtflüchtiges Isocyanat und in ein schwerflüchtiges sekundäres Amin zerfallen, dessen Siedepunkt über dem des Isocyanates und über der Reaktionstemperatur liegt. Die Ausgangsverbindungen sind daher Verbindungen der Formel I, in der R₁ einen geradkettigen, verzweigten oder cyclischen (C₂ - C₁₀) Alkylrest oder einen Benzylrest und R₂ und R₃ einen geradkettigten, verzweigten oder cyclischen (C₄ - C₂₀) Alkylrest oder einen Benzylrest bedeuten. Beispiele für Alkylreste sind ein Ethyl, n-Propyl, i-Propyl, n-Butyl, sek.-Butyl, tert.-Butyl, Hexyl, Octyl, Decyl, Dodecyl, Tetradecyl, Hexadecyl, Octadecyl, oder ein Cyclohexylrest.

Besonders bevorzugte N,N,N'-trisubstituierte Harnstoffe sind daher unter anderem N,N-Dioctyl-N'-ethylharnstoff, N,N-DioctylN'-propylharnstoff, N,N-Dicyclohexyl-N'-propylharnstoff, N,NDioctyl-N'-isopropylharnstoff, N,N-Dibenzyl-N'-butylharnstoff, N,N-Dioctyl-N'-butylharnstoff, N,N-Dioctyl-N'-benzylharnstoff und N,N-Dioctyl-N'-cyclohexylharnstoff.

Die Harnstoffe können zum Beispiel über N-Alkylierung von Harnstoff, wie in EP-OS 0 471 983 beschrieben, hergestellt werden. Die Zersetzung kann in einem unter Reaktionsbedingungen inerten Verdünnungsmittel erfolgen. Als inerte Verdünnungsmittel kommen höhersiedende aliphatische oder aromatische Kohlenwasserstoffe, wie etwa Tetradecan, Dodecan, Hexadecan, Octadecan, Paraffin oder Mischungen derselben in Betracht. Es kann aber auch das Amin, das bei der Zersetzung des Harnstoffes entsteht, als Verdünnungsmittel verwendet werden. Die Reaktionstemperatur liegt je nach verwendetem Harnstoff etwa zwischen 90 - 400 °C, bevorzugt zwischen 150 - 300 °C und besonders bevorzugt zwischen 210 - 280 °C. Die Isolierung der Isocyanate aus dem Reaktionsgemisch erfolgt mittels eines Inertgasstromes, etwa mittels eines Stickstoff- oder Argonstromes. Anschließend werden die Isocyanate entweder in einer Kühlfalle kondensiert oder in einem Lösungsmittel absorbiert. Als Lösungsmittel für die Isocyanate eignen sich aliphatische oder aromatische Kohlenwasserstoffe, die gegebenenfalls auch halogeniert sein können, wie etwa Chloroform, Methylenchlorid, Trichlorethylen, Toluol und Ether wie etwa Tetrahydrofuran. Das Lösungsmitel oder Teile davon können bereits als Dampf mit dem Inertgasstrom oder in die Reaktionsmischung vor Beginn der Zersetzung eingebracht werden.

Die Reaktion kann gewünschtenfalls auch unter reduziertem Druck durchgeführt werden. Die Druckhöhe wird dabei je nach gewählten Ausgangsprodukten und den entstehenden Endprodukten auf den gewünschten Wert eingestellt.

Die Reaktionszeit beträgt abhängig von der Reaktionstemperatur, dem Reaktionsdruck und den Ausgangsverbindungen zwischen 15 und 120 Minuten. Das sekundäre Amin, das bei der Zersetzung im Sumpf bleibt, kann zum Beispiel durch Destillation unter reduziertem Druck aus dem Sumpf isoliert werden und als Ausgangsverbindung für verschiedene Reaktionen eingesetzt werden. Die gewünschten Isocyanate werden in hoher Reinheit und in Ausbeuten bis zu 99,9 % erhalten.

### Beispiel 1:

### Ethylisocyanat

5 g N,N-Dioctyl-N'-ethylharnstof wurden in einer geeigneten Apparatur, welche neben einem thermostatisierten Reaktionsgefäß eine Vorrichtung zum Einführen und günstigenfalls Verteilen des Inertgasstroms in der Reaktionsmischung und eine Kolonne zur Erleichterung der Produktabtrennung (z. B. wurde eine verspiegelte 30 cm-Vigreuxkolonne verwendet) enthält, in 50 g Hexadecan und 2,5 g CHCl₃ 30 Minuten lang auf etwa 250 °C erhitzt.
Das entstehende Ethylisocyanat wurde währenddessen mittels eines Stickstoffstromes (ca. 2 l/h) aus dem Reaktionsgemisch entfernt und in einer mit flüssigem Stickstoff gekühlten Kühlfalle als CHCl₃-Lösung kondensiert.
Ausbeute: 97 %

### Beispiel 2:

### Propylisocyanat

5 g N,N-Dicyclohexyl-N'-propylharnstoff wurden in der im Bsp. 1 beschriebenen Apparatur in 50 g Hexadecan 40 Minuten lang auf etwa 250 °C erhitzt und entstehendes Propylisocyanat mittels Stickstoffstrom aus dem Reaktionsgemisch entfernt und in einer mit flüssigem Stickstoff gekühlten Kühlfalle kondensiert.
Ausbeute: 99,9 %

Folgende Verbindungen wurden analog hergestellt:

| **Produkt** | **Edukt** | **Lösungsmittel** |
|---|---|---|
| 1 i-Propylisocyanat | N,N-Dioctyl-N'-isopropylharnstoff | Hexadecan |
| 2 Butylisocyanat | N,N-Dioctyl-N'-butylharnstoff | Hexadecan |
| 3 | | Paraffin |
| | | Octadecan |
| 4 | N,N-Dibenzyl-N'-butylharnstoff | Hexadecan |
| 5 tert. Butylisocyanat | N,N-Dioctyl-N'-tert.Butylharnstoff | Hexadecan |
| 6 Cyclohexylisocyanat | N,N-Dioctyl-N'-cyclohexylharnstoff | Hexadecan |
| 7 Benzylisocyanat | N,N-Dioctyl-N'-benzylharnstoff | Hexadecan |

## Patentansprüche

1. Verfahren zur Herstellung von Isocyanaten, dadurch gekennzeichnet, daß N,N,N'-trisubstiuierte Harnstoffe der Formel 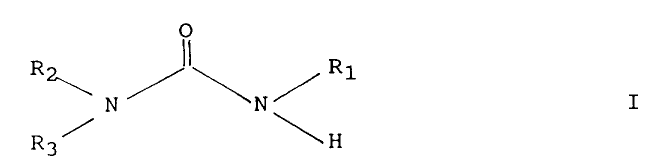 in der R₁ einen geradkettigen, verzweigten oder cyclischen (C₂ - C₁₀) Alkylrest oder einen Benzylrest und R₂ und R₃ einen geradkettigen, verzweigten oder cyclischen (C₄ - C₂₀) Alkylrest oder einen Benzylrest bedeuten in einem unter Reaktionsbedingungen inerten Verdünnungsmittel bei erhöhter Temperatur in ein leichtflüchtiges Isocyanat der Formel R₁NCO und ein schwer flüchtiges sekundäres Amin der Formel R₂R₃NH, dessen Siedepunkt über dem Siedepunkt des Isocyanates und über der angewandten Reaktionstemperatur liegt, zersetzt werden, worauf das Isocyanat mittels eines Trägergasstromes über Kopf abgezogen wird.

2. Verfahren nach Anspruch 1, dadurch gekennzeichnet, daß die Reaktionstemperatur 150 - 300 °C, bevorzugt 220 - 280 °C beträgt.

3. Verfahren nach Anspruch 1, dadurch gekennzeichnet, daß die Harnstoffe unter reduziertem Druck von 0,01 bis 100 mbar zersetzt werden.

## Claims

1. Process for the preparation of isocyanates, characterized in that N,N,N'-trisubstituted ureas of the formula 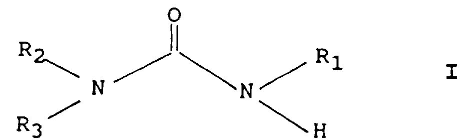 in which Rₗ is a linear, branched or cyclic (C₂-C₁₀)-alkyl radical or a benzyl radical and R₂ and R₃ a linear, branched or cyclic (C₄-C₂₀)-alkyl radical or a benzyl radical, are decompossed, in a diluent which is inert under the reaction conditions, at elevated temperature, to an isocyanate of high volatility of the formula R₁NCO and to a secondary amine of low volatility of the formula R₂R₃NH, whose boiling point is above that of the isocyanate and above the reaction temperature used, after which the isocyanate is drawn off at the top by means of a stream of carrier gas.

2. Process according to Claim 1, characterized in that the reaction temperature is 150 - 300°C, preferably 220 - 280°C.

3. Process according to Claim 1, characterized in that the ureas are decomposed under a reduced pressure of 0.01 to 100 mbar.

## Revendications

1. Procédé de préparation d'isocyanates, caractérisé en ce que des urées N,N,N'-trisubstituées répondant à la formule 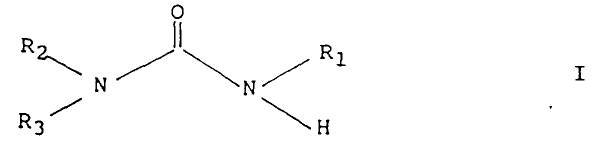 où R₁ signifie un reste alkyle en C₂-C₁₀, linéaire, ramifié ou cyclique, ou un reste benzyle, et R₂ et R₃ signifient un reste alkyle en C₄-C₂₀, linéaire, ramifié ou cyclique, ou un reste benzyle, sont décomposées, dans un diluant inerte dans les conditions de réaction, à une température élevée, en un isocyanate très volatil de formule R₁NCO et en une amine secondaire peu volatile de formule R₂R₃NH dont le point d'ébullition est situé au-dessus du point d'ébullition de l'isocyanate et au-dessus de la température de réaction utilisée, après quoi l'isocyanate est soutiré par le haut de la colonne au moyen d'un flux de gaz porteur.

2. Procédé selon la revendication 1, caractérisé en ce que la température de réaction est de 150 à 300°C, de préférence de 220 à 280°C.

3. Procédé selon la revendication 1, caractérisé en ce que les urées sont décomposées sous une pression réduite de 0,01 à 100 mbar.
